# EUROPEAN PATENT SPECIFICATION

(11) **EP 1 395 670 B1**
(45) Date of publication and mention of the grant of the patent: **31.12.2008**
(21) Application number: 02727803.5
(22) Date of filing: 20.05.2002
(51) Int. Cl.: G01N 33/50, G01N 33/569, B01L 3/00

(54) **DETERMINING ENDOMETRIAL STATUS BY TESTING MENSTRUATION TISSUE**
BESTIMMUNG VON ENDOMETRIALEM STATUS MITTELS PRÜFUNG VON MENSTRUATIONSGEWEBE
PROCEDE DE DETERMINATION DE L'ETAT DE L'ENDOMETRE PAR DES TESTS APPLIQUES AUX TISSUS ENDOMETRIAUX MENSTRUELS

(30) Priority: 21.05.2001 GR 2001100250
(43) Date of publication of application: 10.03.2004
(73) Proprietor: Tsilivakos, Vassilios, 115 25 Athens (GR)
(72) Inventor: Tsilivakos, Vassilios, 115 25 Athens (GR)
(74) Representative: Malamis, Alkisti-Irene
(86) International application number: PCT/GR2002/000031
(87) International publication number: WO 2002/095060

(56) References cited:
- SALAMONSEN LOIS A ET AL: "Endometrial leukocytes and menstruation." HUMAN REPRODUCTION UPDATE., vol. 6, no. 1, January 2000 (2000-01), pages 16-27, XP008008675 ISSN: 1355-4786
- ZHANG JIN ET AL: "Progesterone inhibits activation of latent matrix metalloproteinase (MMP)-2 by membrane-type 1 MMP: Enzymes coordinately expressed in human endometrium." BIOLOGY OF REPRODUCTION, vol. 62, no. 1, January 2000 (2000-01), pages 85-94, XP008008676 ISSN: 0006-3363
- KLENTZERIS LUCAS D ET AL: "Endometrial leukocyte subpopulations in women with endometriosis." EUROPEAN JOURNAL OF OBSTETRICS & GYNECOLOGY AND REPRODUCTIVE BIOLOGY, vol. 63, no. 1, 1995, pages 41-47, XP002197605 ISSN: 0301-2115

## Description

### Technical field of the invention

The present invention refers to the examination of the endometrium. Analysis is performed through tests that utilize endometrial menstruation tissue (period tissue).

### State of the art

Infertility, which is nowadays encountered in large scale in developed countries, is a well-established problem. It is mainly attributable to the age at which today's couples attempt to have children. This age is clearly advanced compared to earlier times, and therefore, not only the woman's hormonal system is getting weaker, but the man's sperm has undergone alterations with time, as well.
Fortunately, the two very important obstacles for achieving fertilization, which are the male factor and the tubal factor (tubal obstruction) have been conquered, not so much due to therapies for the improvement of sperm, but to a higher degree due to the techniques of spermatenchysis and in vitro fertilization (IVF). IVF is the only technique that overcomes tubal obstruction problems.
However, despite the establishment of these methods worldwide, results are not as successful as expected. The success rates are 5-40% as far as implantation is concerned, whereas there is a high percentage of fetal rejection following IVF assisted conception.

The endometrium is a solid tissue, covering the uterus from the inside and is influenced by the periodical action of hormones. In a 28-day cycle starting from the 1^{st} day of menstruation, the endometrium increases in thickness and the glands that it contains gain length, under exposure to estrogens. The endometrium functionalis, which is shed and newly formed during each cycle, is developing on the endometrium basalis, which lies in the deeper level.
Ovulation takes place in the ovaries on the 14^{th} day of the cycle. Then, the corpus luteum, which is the remaining part of that follicle that has freed the oocyte, produces progesterone. Progesterone prepares the endometrium, which is already developed, for the implantation of the fetus. Towards the end of the cycle, due to the degeneration of the corpus luteum, which ceases to function, the endometrium functionalis does not receive the reinforcing activity of progesterone and falls (menstruation). The cellular composition of the endometrium undergoes circular changes due to hormonal activity, whose ultimate goal is implantation as well as preservation of the fetus (once fertilization has occurred).
It is noteworthy to be pointed out, that menstruation tissue is not blood as it was traditionally believed. Menstruation material does not coagulate, whilst the proportion of white blood cells it contains is totally different from that of the blood. The large amount of red blood cells which give the impression of blood, are situated in the premenstrual endometrium outside of the vessels and consequently are released with the menstrual tissue, along with the rest of the endometrial cells. Thus, the notion of "menstrual tissue" corresponds to the endometrium at the last day of the cycle.

### Technical issue

In order for fertilization to take place and for the fetus to be preserved, certain conditions are required. These conditions render the endometrium the only organ that can preserve foreign (non-self) antigens, in contrast to the laws of human physiology (immunology), which commands the rejection of foreign grafts. Unfortunately, it has not been possible to understand how the endometrium is an exception to the worldwide laws at that point. However, the presence of infections (particularly those due to mycoplasmas), as well as immunopathological factors, have been implicated at various degrees in preventing the implantation or the preservation of the fetus, that is if there are no chromosomal defects or other evident reasons leading to fetal rejection.

By having studied 750 women with history of infertility and 500 women with history of miscarriages using various techniques, we have come to the conclusion that specific cells of the immune system, which colonize the endometrium play a pivotal role in the process of implantation, and in the preservation of the fetus. Furthermore, we have come to the conclusion that toxic white blood cells that migrate to the endometrium, possibly in an attempt to extinguish microorganisms, are forming an inappropriate microenvironment both for implantation, and preservation of the fetus within the endometrium.

Zhang et al., Biol. Reprod., 2000, 62(1), p. 85-94, analyzes the expression and localization of proMMP-2, MT1-MMP, and MT2-MMP in human endometrium across the menstrual cycle.

Salamonsen et al., Hum. Reprod. Update 2000, 6(1), p. 16 to 27 reviews evidence that menstruation occurs as a result of an inflammatory process and describes that neutrophils are localized in areas of endometrial breakdown in patients treated with high dose oral progestins or implanted levonorgestrel. It is also described that CD45RA+ B lymphocytes are also detected in low numbers during the entire cycle and are present in peri-menstrual tissue.

### Description of the invention

As a result of the abovementioned, we consider it purposeful and useful to collect fallen endometrial tissue (menstruation tissue) and to utilize it in order to analyze the status of the endometrium. This examination may be performed on a routine basis with the purpose of:
A) Studying the composition of the subpopulations of endometrial lymphocytes.
B) Studying the presence of microorganisms in the endometrium.
C) Studying the cellular composition of the endometrium (which is useful for various diagnostic purposes).
D) Simultaneous analysis of the composition of the subpopulations of lymphocytes and detection of microorganisms.

Unfortunately, nowadays throughout the world the hostility of the endometrium, which reflects the chances of conception following successful fertilization, is underestimated.
According to our opinion, the vast majority of the unsuccessful attempts can be attributed to the hostility of the endometrium. This concept is sustained by the fact that in most attempts the implantation is not successful, even when sperm or oocyte originating from fertile donors is used.

Currently, the study of the endometrium is approached by the following five ways:
1) **Endovaginal ultrasonography.** This method determines endometrial thickness, which reflects its response to estrogens. This indication is taken into consideration for the design of IVF protocols, as well as for determining the time of ovulation. However, this examination has limited applications, since it does not yield any information on the receptiveness of the endometrium, on the presence of an inflammation, on the risk of fetal rejection etc.
2) **Uterosalpingography.** This examination is used to screen the cavity of the uterus and depicts tubal permeability. Endometrial polypodes can also be visualized. However, this method yields no information on the suitability of the endometrium (as a microenvironment) for the implantation of the fetus.
3) **Hysteroscopy.** This is a visual macroscopic method of surveying the surface of the endometrium. One can detect polypodes, morphological defects, symphysis of the endometrium, and even inflammations, as long as these have caused macroscopic lesions. However, one cannot study the microenvironment in order to determine how hostile the endometrium is for accepting and preserving (developing) the fetus. This examination is characterized by its relatively high cost and it requires anesthesia.
4) **Endometrial biopsy.** This is a particularly useful examination, since it can be used to determine the phase of the cycle, the time of ovulation during the current cycle, the inadequacy of the corpus luteum and even the presence of microbes through molecular analysis of the tissue. Important and valuable information regarding the endometrial immunological status have been obtained from examinations of biopsy material, since this is the only method to date that allows us to determine the cellular composition of the endometrium and to draw conclusions about factors leading to infertility or fetal rejection. The material is extracted by a needle or by curettage following anesthesia. The disadvantage of this method is that it is relatively painful and it cannot be performed repeatedly. Besides, there is a danger of injury if the needle's pinch is not accurate and therefore, the doctor performing the extraction must be highly specialized. Additionally, it is not possible to conduct a full immunological study of the endometrium since the tissue is fixated in formalin and undergoes such a treatment, that many molecules of the lymphocytes are altered and cannot be recognized by routine methods (immunohistochemistry), as a result conclusions cannot be drawn.
**5) Cultivation of cervical** fluid. This is a very important conventional examination used to diagnose infections of the genital tract. It is highly sensitive in detecting infections of the vagina and the cervix, yet does not seem to be equally sensitive for diagnosis of endometrial inflammations. The microbes leading in infertility have an ascending direction, showing a tropism towards the endometrium and the fallopian tubes, thus there is an increased likelihood and as a matter of fact is a very frequent phenomenon, for the culture of cervical fluid to turn out negative, despite the existence of microbial agent in the endometrium.
   There have been attempts to detect microbes by molecular DNA tests (polymerase chain reaction, PCR) by using extracted cervical fluid. No difference has been reported regarding the sensitivity of the two ways of detection (cultivation of cervical fluid versus examination of menstruation tissue). Therefore, the sensitivity of cultivation as a method is not disputed, however the problem lies in that site of extraction is not suitable. It should be noted that the location where the liquid for this cultivation is extracted from is more than 5 cm away from the site of fetal implantation.

### Presentation of our method.

In order to investigate infertility or the risk of fetal rejection, menstrual tissue samples are examined to determine the presence of microorganisms in the endometrium and the cellular composition of the endometrium.

### Method of carrying out the invention:

During the phase of the first major menstrual flow; the woman under examination places a quantity of 100-1000µl from the fallen endometrium (menstruation tissue) of the previous cycle in a 50-ml tube with a wide opening, which contains a small quantity of an isotonic buffer (that maintains pH stability and secures viability of the cells),
Menstruation flow is automatic and involuntary, no special efforts are required on the woman's side and an interval ranging from of 2 - 15 minutes is sufficient for material collection.

In detail: the woman, while in a seated position, places the opening of the tube, without the cover, at the back limit of the vulva and the drops fall freely within the tube. Depending on the quantity of the flow, the material is falling towards the bottom due to gravity and is mixed with the buffer. In case that, due to high viscosity (presence of mucus etc.), the drop of the tissue stops at the opening or at the wall of the tube, then the woman needs only to put the cover back on the tube and flick upside-down couple of times, so that the buffer's drop draws the specimen to the bottom of the tube.

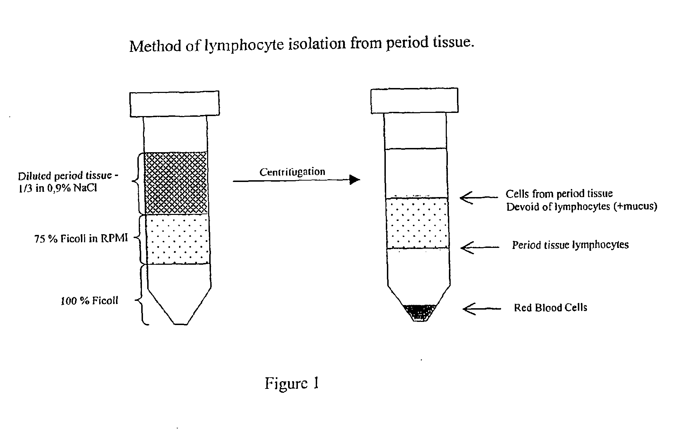

The endometrial inspection, in the context of investigating infertility or the risk of rejection of a fetus, according to one application of this invention may request the study of the endometrial lymphocytes with a specialized immunophenotypic analysis. In this case, the isolation of lymphocytes from the specimen is rather easy following well-established methods, and their study from that point onwards may be performed according to methods, which have already been described for the examination of other corresponding cell populations from different body fluids (blood, pleural fluid, etc.).
More specifically, as shown on the left part of Figure 1, the extraction of lymphocytes from the specimen is performed with double-layered Ficoll (First layer 100% Ficoll and 75% RPMI in the second). The sample is diluted in a triple volume of normal sterile saline solution (0.9% NaCl), and is placed on the top of the double-layered ficoll, forming strata. Centrifugation follows for 45 min at 900 rpm and then the lower cell layer is isolated as shown at the left part of Figure 1. Two (2) washes in phoshate buffered saline (PBS) follow. Finally, the lymphocytes are labeled and analyzed by flow cytometry.
Collection of endometrial tissue may be easily performed at the woman's house but the specimen must be brought within 5-6 hours from collection. It should be clarified that the specimen must be collected within the first 30 hours of menstruation, since in order to carry out the above examination a sufficient quantity of lymphocytes are required.

Another application of the invention, in order to examine the endometrium may involve studying the presence of microbes (i.e. mycoplasmas, chlamydia etc.) or other microorganisms. In this case, the sample collection may take place at any phase of the menstruation days. Each woman may collect material at home and bring it within a longer time period (up to one (1) week) for examination through DNA tests for the detection of microorganisms. In other words even if menstruation starts on a public holiday, the woman under examination has the tube, collects the material and sends it by mail, thus it is very convenient in cases where she resides far from the laboratory (in the province, island etc.). This method of detecting microbes causing gynecological problems can be carried out not only for investigating the microenvironment of fetal implantation and identifying the problem, but also as a test prior any attempts to have children, thus preventing any future reproductive failure. The importance lies to the fact that microbial infections in young age, such as chlamydial or mycobacterial infections can potentially lead to tubal obstruction or hydrosalpinx. The detailed description, which follows, will indicate this method of examination for the presence of microbes as by far more sensitive than the vaginal fluid cultivation or the study of cervical fluid in general. Further, the advantages of our method for the examination of microorganisms in virgo women are obvious.

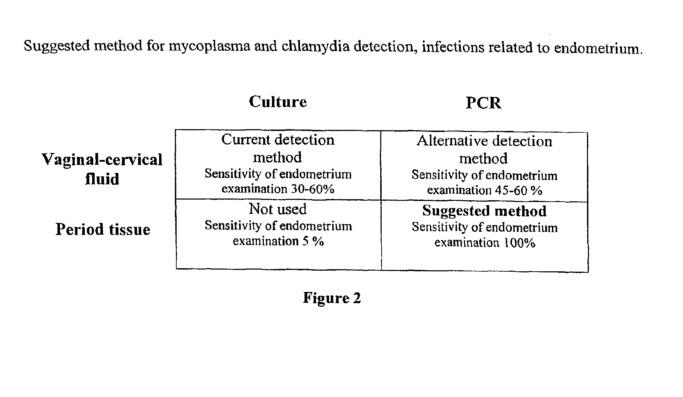

Figure 2 shows the sensitivity of the proposed examination for detecting microbial infections related to endometrium. The cultivation of cervical fluid as a conventional method of detecting endometrial microbes is 30-60%. Molecular detection (e.g. PCR) performed on cervical fluid as an alternative method for detecting endometrial microbes is 45-60% sensitive. The cultivation of menstruation tissue is not used to screen for endometrial microbes and is less than 5% sensitive. On the other hand, the proposed methodology of molecular detection (PCR) on menstruation tissue is 100% sensitive for screening the endometrium for the presence of microbes.

Another application of the invention is the examination of the cellular composition of the endometrium, which may be requested for diagnostic purposes (i.e. unovulatory cycle).

### Advantages of our method

As far as the advantages of infertility status determination using menstruation tissue specimens in their natural state are concerned, the following need to be stressed out:
It is easy to extract sufficient quantity of material: In this aspect, this method is superior compared to other techniques. The woman avoiding any gynecological examination stress can collect the sample alone, at her home and repeat it if the first attempt fails. Besides, with the help of the scale existing at the bottom part of the tube, the woman is able to monitor the quantity of the sample. The quantity taken (100 - 1000 µl) suffices for all the examinations that follow. It is particularly advantageous compared to endometrial biopsies, as far as information regarding the examination or even the prevention of infertility are concerned.
Low cost: The special collection tube containing the buffer has a very low cost, which ranges from 1%-0,5% of the cost of endometrial biopsies.
Sensitivity of the test (validity of the result): Having examined (at the laboratory LOCUS MEDICUS Ltd. in Athens) approximately 200 women with a history of infertility and/or miscarriages, by screening for mycoplasmas and chlamydia in the menstruation tissue samples, we have observed the following:
   Out of a total number of 140 women, who had recently been subjected to an examination of cervical fluid without detecting the aforementioned microbes, 44% of them yield a positive result for *Mycoplasma hominis* (MH), 32% of them were found positive for *Ureaplasma urealyticum* (UU), while 46% of them were positive for *Chlamydia trachomatis* (CT). This means that the cases that had been found negative to MH by cultivation of the cervical fluid, actually carried the microbe since when using the proposed method of testing (in drops of menstruation tissue) 44% of the women examined were positive. Equally, among these women whose cervical fluid was found negative for UU, 32% yielded a positive result by our methodology.

Considering that all these microbes play a very important role in the pathophysiology of infertility and also that they are basic factors for miscarriage, the decisive importance of the examination of menstruation tissue is obvious, since this examination clearly indicates whether the site of fetal implantation is infected or not by dangerous microorganisms.
As far as chlamydial infections are concerned, the percentage of infection that has been detected by menstruation tissue examination was 46% of the cases that have been found negative with conventional tests.
The presence of chlamydia is directly associated with tubal obstruction, and since this infection is particularly common at the ages of 20-25 years, the need to examine the endometrium for chlamydia for preventive reasons is essential, even before a woman decides to have children.

The molecular detection of microorganisms on menstruation samples is a much more sensitive method, compared to the molecular detection of the same microorganisms in a fine needle biopsy samples. The advantage lies on the fact that in the menstruation tissue consists of representative material from the whole mass of the endometrium, which of course is not the case for biopsy material, that is taken locally from one point only.

Additionally during immunophenotypic analysis by flow cytometry, the molecules of the lymphocytes remain unaltered and therefor detectable because the isolation process allows the cells of interest to be fully viable.
At this point, our method is by far superior to immunophenotyping of fixed biopsy material. The reason is that formalin, the fixing compound used for the preservation of biopsy material even thought it prevents the structural decomposition of the fixed material, it changes its molecular structure. In other words, what one obtains through biopsy is material for observation, which does not allow the application of a large range of tests. An additional use of biopsy material is DNA extraction for microbial investigation. Instead, using our method we can proceed to the functional analysis of the material from the endometrium and not only its observation.
Reproducibility of the method: A very important advantage of our method is that it can be repeated and thus therapies results monitored. Both immno-therapeutic treatments, which aim at reducing the supply of toxic cells in the endometrium, as well as antibiotic treatments, which aim at extinguishing microbial agents, may be monitored in each woman under examination. It is particularly important that the method is characterized by high sensitivity and specificity, which minimize false negative and false positive results, respectively. In few words, after an antibiotic treatment, a new examination can take place and the absence of the microbe can be confirmed and actually trust the negative result.

Synchronization of the endometrium: Investigating the cellular elements of the last day of the cycle is safely performed with use of our method. Women have sometimes irregular cycles, the results of other methods are not synchronized among women. When endometrial biopsy is carried out, since the last day of the cycle cannot be determined, the degree of endometrial removed cannot be estimated.
In essence with our method, which is the object of the present invention, novel ways of studying infertility may be introduced, based on the composition of the lymphocytic population in the final phase of the monthly cycle, and therapy results evaluation. There is no other way to approach this issue, while it is obvious that such measurements comprise important criteria for a woman to be able to proceed to an IVF cycle with good probabilities of success.

### An example of an application of the method:

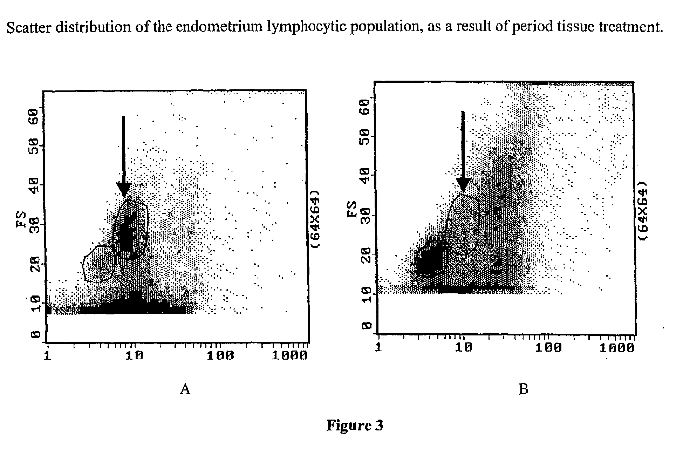

Figure 3 shows the distribution of the lymphocytes of menstrual tissue based on their size (vertical axes) and granulation (horizontal axes), when these are taken from a fertile woman (Figure 3A) and an infertile woman (Figure 3B).
The cells within the circle, shown by the arrow in each of the Figures 3A and 3B are vital for a woman in order to receive and preserve the fetus. These cells, which are NK (Natural Killers) lymphocytes (CD56⁺CD16⁻CD3⁻) maintain the "friendliness" of the endometrium towards the fetus, and must account for more than 60% of the total lymphocytes. The total amount of the lymphocytes are the cells shown within the two circles of Figures 3A and 3B
It is obvious that the presence of these cells is significantly reduced in the endometrium of women with infertility. Following proper treatment (e.g.antibiotics), which lasts at least two menstrual cycles, the percentage of these cells may rise and then the woman increases the chances of fetal implantation and preservation, according to our own unpublished observations.
These cells are also reduced in the peripheral blood of infertile women. However, in the endometrium the proportion of these cells does not appear to be affected by other factors, such as infections of the respiratory tract, urinary tract infections etc. The cells within the small circle of Figures 3A and 3B are lymphocytes that resemble those of the blood. They include toxic populations, which although can protect the endometrium from infections, however (some of these, approximately half of them) are toxic to the fetus. What is depicted in Figures 3A and 3B is the first presentation worldwide of the specific application of the method, which constitutes the object of the present invention.
Following examinations of menstruation tissue samples, we consider it meaningful for women to attempt IVF or spermatenchysis only when their sample image is similar to that of the normal sample shown, in Figure 3^{A}, i.e. if there are enough endometrial type NK, which are shown by the arrow.

## Claims

1. Method of examining the endometrium, comprising analysing a sample of endometrial menstruation tissue obtainable by collecting menstrual flow material, where the menstruation tissue is analysed to study the presence of microorganisms in women that are sexually active and also in virgo women.

2. Method of examining the endometrium, comprising analysing a sample of endometrial menstruation tissue obtainable by collecting menstrual flow material, where the menstruation tissue is analyzed for the simultaneous study of the composition of the subpopulation of lymphocytes and the presence of microorganisms.

3. Method according to claims 1 or 2, where the menstruation tissue is analyzed to study the presence of chlamydia, mycoplasma and ureoplasma.

4. Method of examining the endometrium, comprising analysing a sample of endometrial menstruation tissue obtainable by collecting menstrual flow material, where the examination of the endometrium takes place for monitoring the result of immunotherapy or of antibiotic treatment.

5. Method according to any one of claims 1 to 4, where a sample of between 100 to 1000 µl of the menstruation tissue is taken during the 30 first hours of menstrual flow and right after its collection it is mixed with an isotonic buffer which secures stability of pH and viability of the cells, and where the study of the sample takes place within 5-6 hours from extraction, at the latest.

6. Method according to any one of claims 1 to 4, where a sample of 100 to 1000 µl of the menstruation tissue is taken at any stage of the menstrual flow and right after its collection it is mixed with an isotonic buffer which secures stability of pH and viability of the cells, and where the examination of the sample takes place within 1 week from the extraction.

## Patentansprüche

1. Verfahren zum Untersuchen des Endometriums, umfassend das Analysieren einer Probe von endometrialem Menstruationsgewebe, das durch Sammeln von Material des Menstruationsflusses erhalten werden kann, wobei das Menstruationsgewebe analysiert wird, um das Vorliegen von Mikroorganismen in Frauen, die sexuell aktiv sind, und auch in Jungfrauen zu untersuchen.

2. Verfahren zum Untersuchen des Endometriums, umfassend das Analysieren einer Probe von endometrialem Menstruationsgewebe, das durch Sammeln von Material des Menstruationsflusses erhalten werden kann, wobei das Menstruationsgewebe für die gleichzeitige Untersuchung der Zusammensetzung der Subpopulation von Lymphozyten und des Vorliegens von Mikroorganismen analysiert wird.

3. Verfahren gemäß den Ansprüchen 1 oder 2, wobei das Menstruationsgewebe analysiert wird, um das Vorliegen von Chlamydien, Mycoplasmen und Ureaplasmen zu untersuchen.

4. Verfahren zum Untersuchen des Endometriums, umfassend das Analysieren einer Probe von endometrialem Menstruationsgewebe, das durch Sammeln von Material des Menstruationsflusses erhalten werden kann, wobei die Untersuchung des Menstruationsgewebes zum Überwachen des Ergebnisses einer Immuntharapie oder einer antibiotischen Behandlung stattfindet.

5. Verfahren gemäß einem der Ansprüche 1 bis 4, wobei eine Probe von 100 bis 1000 µl des Menstruationsgewebes während der ersten 30 Stunden des Menstruationsflusses genommen wird und unmittelbar nach ihrer Gewinnung mit einem isotonischen Puffer, der die Stabilität des pH-Werts und die Lebensfähigkeit der Zellen sicherstellt, gemischt wird und wobei die Untersuchung der Probe spätestens innerhalb von 5-6 Stunden nach der Entnahme stattfindet.

6. Verfahren gemäß einem der Ansprüche 1 bis 4, wobei eine Probe von 100 bis 1000 µl des Menstruationsgewebes in irgendeinem Stadium des Menstruationsflusses genommen wird und unmittelbar nach ihrer Gewinnung mit einem isotonischen Puffer, der die Stabilität des pH-Werts und die Lebensfähigkeit der Zellen sicherstellt, gemischt wird und wobei die Untersuchung der Probe innerhalb einer Woche nach der Entnahme stattfindet.

## Revendications

1. Procédé d'examen de l'endomètre, comprenant l'analyse d'un échantillon du tissu endométrial menstruel pouvant être obtenu en recueillant le matériau du flux menstruel, dans lequel le tissu menstruel est analysé pour étudier la présence de micro-organismes chez des femmes qui sont sexuellement actives et également chez des femmes vierges.

2. Procédé d'examen de l'endomètre, comprenant l'analyse d'un échantillon du tissu endométrial menstruel pouvant être obtenu en recueillant le matériau du flux menstruel, dans lequel le tissu menstruel est analysé pour l'étude simultanée de la composition de la sous-population des lymphocytes et la présence de micro-organismes.

3. Procédé selon les revendications 1 ou 2, dans lequel le tissu menstruel est analysé pour étudier la présence de chlamydia, mycoplasme et uréoplasme.

4. Procédé d'examen de l'endomètre, comprenant l'analyse d'un échantillon du tissu endométrial menstruel pouvant être obtenu en recueillant le matériau du flux menstruel, dans lequel l'examen de l'endomètre est effectué pour contrôler le résultat d'une immunothérapie ou d'un traitement antibiotique.

5. Procédé selon l'une quelconque des revendications 1 à 4, dans lequel un échantillon d'entre 100 à 1000µl du tissu menstruel est prélevé au cours des 30 premières heures du flux menstruel et juste après son prélèvement, il est mélangé avec un tampon isotonique qui assure la stabilité du pH et la viabilité des cellules, et dans lequel l'étude de l'échantillon est effectuée dans les 5 à 6 heures de l'extraction, au plus tard.

6. Procédé selon l'une quelconque des revendications 1 à 4, dans lequel un échantillon de 100 à 1000 µl du tissu menstruel est prélevé à n'importe quel stade du flux menstruel et juste après son prélèvement, il est mélangé avec un tampon isotonique qui assure la stabilité du pH et la viabilité des cellules, et dans lequel l'examen de l'échantillon est effectué dans la semaine de l'extraction.
